(19) **Europäisches Patentamt**
**European Patent Office**
**Office européen des brevets**

(11) **EP 3 254 624 B1**

(12) **EUROPEAN PATENT SPECIFICATION**

(45) Date of publication and mention
of the grant of the patent:
**09.01.2019 Bulletin 2019/02**

(51) Int Cl.:
***A61B 6/03*** *(2006.01)* ***A61B 6/00*** *(2006.01)*

(21) Application number: **17158686.0**

(22) Date of filing: **01.03.2017**

(54) **METHOD AND APPARATUS FOR ANALYZING NUCLEAR MEDICINE IMAGE OF MYOCARDIA**

VERFAHREN UND VORRICHTUNG ZUR ANALYSE EINER NUKLEARMEDIZINISCHEN AUFNAHME DES MYOKARDS

PROCÉDÉ ET APPAREIL D'ANALYSE D'IMAGE DE MÉDECINE NUCLÉAIRE DE MYOCARDE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **08.06.2016 JP 2016114251**

(43) Date of publication of application:
**13.12.2017 Bulletin 2017/50**

(73) Proprietor: **Nihon Medi-Physics Co., Ltd.**
**Tokyo 136-0075 (JP)**

(72) Inventors:
• **Nishida, Kazumasa**
**Koto-ku, Tokyo 1360075 (JP)**

• **Hamada, Kazuo**
**Koto-ku, Tokyo 1360075 (JP)**
• **Kobayashi, Kazunori**
**Koto-ku, Tokyo 1360075 (JP)**

(74) Representative: **V.O.**
**P.O. Box 87930**
**Carnegieplein 5**
**2508 DH Den Haag (NL)**

(56) References cited:
**JP-B1- 5 754 718     US-A1- 2011 293 519**

• **STEPHAN NEKOLLA ET AL: "The effect of different normalizations for the quantification of myocardial F-18 FDG uptake", J NUCL MED, vol. 56, 1 May 2015 (2015-05-01), XP055397666,**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

FIELD OF THE INVENTION

The present application relates to a method of analyzing myocardial nuclear medicine image data and an apparatus for analyzing myocardial nuclear medicine image data.

DESCRIPTION OF THE RELATED ART

Nuclear medicine technology is used to yield various types of physiological and biochemical information about the heart in many cases. Specifically, a single-photon emission computed tomography (SPECT) examination has excellent characteristics including an easy load examination, a high examination success rate, low invasiveness, and no load on kidney functions.

The primary image obtained from nuclear medicine measurement is prepared by the imaging of radiation count values or tissue radioactivity concentrations. Pixels corresponding to the position at which a tracer is highly accumulated have a large pixel value and are displayed brightly. However, the radiation count value or the tissue radioactivity concentration is affected by various factors, and thus even when particular pixels have a pixel value different from those of the other positions, whether the corresponding tissue is abnormal is not necessarily evident. To address this uncertainness, attempts have been made to normalize pixel values in accordance with a certain rule so as to enable quantitative evaluation of the pixel values. As such a quantitative value, a standardized uptake value (SUV) is typically used. The SUV is determined in accordance with the following formula:

$$\text{SUV} = \text{Tissue radioactivity concentration} / \{\text{Administered}$$

$$\text{radiation dose} / \text{Body mass of subject}\}.$$

In other words, the SUV is calculated by normalization of a tissue radioactivity concentration by the administered radiation dose per body mass. In place of a simple body mass, a lean body mass is used in some cases (Non-Patent Literature 1).

[Related Art Documents]

[Non-Patent Literature]

[Non-Patent Literature 1] Yoshifumi Sugawara, Kenneth R. Zasadny et al., "Reevaluation of the Standardized Uptake Value for FDG: Variations with Body Weight and Methods for Correction", November 1999 Radiology, 213, 521-525.
[Non-Patent Literature 2] Stephan Nekolla ET AL: "The effect of different normalizations for the quantification of myocardial F-18 FDG uptake", J Nucl Med, vol. 56, 1 May 2015 (2015-05-01), XP055397666 discloses using LBM (lean-body-mass) and BSA (body-surface-area) for calculating SUV for PET imaging using F-18 FDG.

[Patent Literature]

JP 5754718 discloses using "bone mineral content" and "bone mineral density" for obtaining normalized nuclear medicine image data acquired using a bone disease diagnostic agent.

SUMMARY OF THE INVENTION

The existing SUV is determined on the assumption that a tracer is evenly distributed in the whole body or muscle. In the case of the nuclear medicine examination of the heart, a tracer is, however, accumulated mainly in myocardia, and thus the assumption of the existing SUV may be inappropriate. On this account, there is a demand for a novel technique for quantitatively evaluating tracer accumulation.

An embodiment of the invention described in the present application is intended to normalize image data obtained from myocardial nuclear medicine measurement, using a value relating to the size of the heart.

In a preferred embodiment, the pixel value of each pixel of the myocardial nuclear medicine image data is converted into an SUV represented by the following formula:

$$\text{SUV} = \text{Tissue radioactivity concentration} /$$

$$(\text{Administered radiation dose} / \text{Value relating to size of}$$

$$\text{heart}).$$

The invention uses a value relating to the size of the heart in which a tracer is accumulated, as a normalization standard to normalize myocardial nuclear medicine image data. The normalization standard thus reflects actual conditions of a cardiac function more correctly than in the related art. This improves the validity of a normalized value as compared with the related art and enables more appropriate image evaluation than ever.

In the invention, the "value relating to the size of the heart" may be a heart weight, for example. The heart weight may be a myocardial weight, for example. The myocardial weight may be a value obtained by multiplying a myocardial volume by a density factor, for example.

In the invention, the "tissue radioactivity concentration" may be a value obtained by multiplying a pixel value of the myocardial nuclear medicine image data by a becquerel calibration factor (BCF). The BCF is a factor for converting a radiation count value into a radioactivity concentration (for example, Bq/ml). The BCF can be determined by a known method. For example, a nuclear medicine image of a vial (or a syringe) containing a radiopharmaceutical agent having a known total radioactivity can be taken, and the BCF can be calculated in accordance with the following formula:

$$\text{BCF} = \text{Decay-corrected total radioactivity (Bq)} /$$

$$(\text{Total count of all slices} / \text{Collection time (seconds)}).$$

To determine the BCF from the data obtained using a cylindrical phantom, the following formulas may be used:

$$\text{Volume factor} = \text{Average count value per slice} /$$

$$(\text{Volume of single pixel} \times \text{Collection time (seconds)})$$

$$\text{BCF} = \text{Decay-corrected total radioactivity (Bq)} /$$

$$(\text{Phantom volume} \times \text{Volume factor}).$$

In some embodiments, the BCF may be subjected to collection time correction. The collection time correction may be performed by multiplying {Volume of single pixel [cm$^3$] / Collection time [sec]} by BCF, for example.

In some myocardial nuclear medicine image data, each pixel value itself may represent a radioactivity concentration. Needless to say, no BCF is needed in such a case.

An embodiment of the invention includes the following method. The method is for processing myocardial nuclear medicine image data and is performed through execution of a program instruction by processing means in an apparatus.

The method includes operating the apparatus as first means for storing a heart parameter serving as a value relating to a size of a heart and as second means for storing an administered radiation dose.

This method also includes converting pixel values of at least part of pixels of the image data using the values stored in the first means and the second means into SUVs in accordance with the following formula, and storing the SUVs:

$$\text{SUV} = \text{Tissue radioactivity concentration} /$$

$$(\text{Administered radiation dose} / \text{Value based on heart}$$

$$\text{parameter}).$$

In some embodiments, the heart parameter is a myocardial weight, and the value based on the heart parameter is also a myocardial weight.

In some embodiments, the heart parameter is a myocardial volume, and the value based on the heart parameter is a myocardial weight calculated by multiplying the myocardial volume by a conversion factor.

An embodiment of the invention includes a computer program including a program instruction configured to cause an apparatus to perform the above-described method when the computer program is executed by processing means in the apparatus.

Another embodiment of the invention includes an apparatus including processing means and memory means. The memory means stores a program instruction, and the program instruction is configured to perform the above-described method when the program instruction is executed by the processing means.

Some embodiments of the invention of the present application thought to be preferred now are specified by the appended claims. However, the configurations specified by these claims do not necessarily completely encompass all the novel technical spirit disclosed in the description and the drawings of the present application. It should be noted that the applicant claims a right to the patent of all the novel technical spirit disclosed in the description and the drawings of the present application regardless of whether the technique is described in the present claims.

BRIEF DESCRIPTION OF THE DRAWINGS

FIG. 1 is a diagram for explaining a hardware configuration of a system capable of performing the present invention; and

FIG. 2 is a flowchart for explaining a preferred example of SUV conversion processing for myocardial nuclear medicine images.

DETAILED DESCRIPTION OF THE PREFERRED EMBODIMENTS

Preferred embodiments of the technical spirit disclosed in the present application will now be described with reference to the attached drawings.

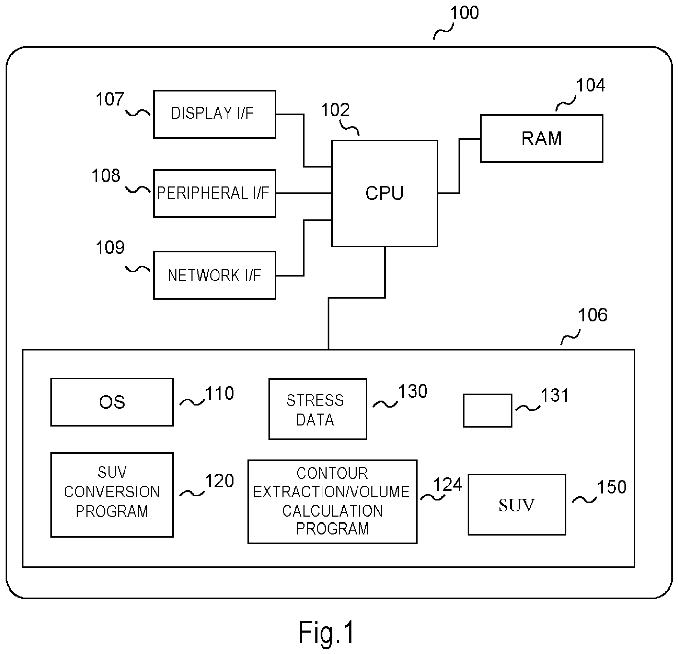

FIG. 1 is a diagram for explaining a hardware configuration of a system 100 capable of performing the present invention. As illustrated in FIG. 1, the hardware configuration of the system 100 is substantially the same as those of conventional computers, and can include a CPU 102, a main memory unit 104, a mass storage unit 106, a display interface 107, a peripheral interface 108, and a network interface 109, for example. Similarly to conventional computers, the main memory unit 104 may be a high-speed random access memory (RAM), and the mass storage unit 106 may be an inexpensive, large-capacity hard disk or SSD. The system 100 may be connected to a display for displaying information via the display interface 107. The system 100 may also be connected to user interfaces, such as a keyboard, a mouse, and a touch panel, via the peripheral interface 108. The network interface 109 can be used to connect the system to other computers and the Internet via a network.

The mass storage unit 106 stores an operating system (OS) 110, an SUV conversion program 120, an alignment program 122, and a contour extraction/volume calculation program 124. The most basic function of the system 100 is provided through execution of the OS 110 by the CPU 102. The SUV conversion program 120 includes program instructions relating to the novel processing disclosed in the present application. Through execution of at least part of these instructions by the CPU 102, the system 100 can perform the novel processing disclosed in the present application.

The contour extraction/volume calculation program 124 includes instructions for extracting the myocardial contour. Some algorithms and software for myocardial contour extraction are known, and such an algorithm is disclosed by the present applicant in PCT International Publication (WO2013/047496A1), for example. In addition, QGS by Cedras-Sinai Medical Center, 4D-MSPECT by the University of Michigan, and pFAST by Sapporo Medical University are also disclosed as the algorithm or software for myocardial contour extraction. The program instructions included in the contour extraction/volume calculation program 124 may be configured to extract the myocardial contour using such an algorithm or software and to calculate the volume of the extracted myocardium. An embodiment of the invention disclosed in the present application can be operated together with various myocardial contour extraction algorithms, but the algorithm described in WO2013/047496A1 is preferably used to extract the myocardial contour because the algorithm has high extraction accuracy.

The mass storage unit 106 can further store three-dimensional nuclear medicine image data 130. Such nuclear medicine image data is to be analyzed or operated by the programs 120 and 124. The mass storage unit 106 can also store a collection condition file 131 that stores various data collection conditions relating to the nuclear medicine image data. The mass storage unit 106 can further store SUV data 150 prepared by converting the nuclear medicine image data 130 by the SUV conversion program 120.

The system 100 can also include typical components included in a common computer system, such as a power supply and a cooler, in addition to the units illustrated in FIG. 1. Known embodiments of the computer system can include various forms using various techniques such as distribution, redundancy, and virtualization of memory units, use of multiple CPUs, CPU virtualization, use of a processing-specific processor such as a DSP, and a combination of hardware for particular processing performed by a CPU. The invention disclosed in the present application can be installed on any computer system, and the type of computer system does not limit the scope of the invention. The technical spirit disclosed in the present description can be typically embodied as (1) a program including instructions configured to cause an apparatus or a system including processing means to perform various types of processing described in the present description when the program is executed by the processing means; (2) a method of operating an apparatus or a system implemented by the processing means executing the program; or (3) an apparatus or a system including the program and processing means configured to execute the program, for example. As described above, software processing may be partially made into hardware.

It should be noted that the data 130, 131, or 150, for example, is not stored in the mass storage unit 106 in many cases while the system 100 is being produced and sold or is being started. Such data may be transferred from an external device to the system 100 via the peripheral interface 108 or the network interface 109, for example. In some embodiments, the data 131 and 150 may be formed through execution of the SUV conversion program 120 by the CPU 102. Depending on an installed alignment program 122 or an installed OS 110, at least one of the data 131 and 150 is not stored in the mass storage unit 106 but is stored only in the main memory unit 104 in some cases. It should be noted that the scope of the invention disclosed in the present application is not limited by whether the data is included.

Next, the three-dimensional nuclear medicine image data 130 will be described in detail. The image data is obtained by nuclear medicine measurement performed on a myocardium as the tissue to be examined. In the present example, the three-dimensional nuclear medicine image data is obtained using SPECT as a nuclear medicine measurement technique. The SPECT examination of a myocardium as the tissue to be examined includes a myocardial blood flow SPECT examination to detect ischemia, for example. Known SPECT radiopharmaceutical agents suitable for the examination are $^{201}$TlCl (thallium chloride) injection solution, technetium ($^{99m}$Tc) tetrofosmin injection solution, and 15-(4-iodophenyl)-3(R,S)-methylpentadecanoic acid ($^{123}$I) injection solution, for example.

In the examples specifically described below, the image data 130 is image data in which each pixel value corresponds to a radiation count value. In some embodiments, the image data 130 may be image data in which each pixel value represents a tissue radioactivity concentration.

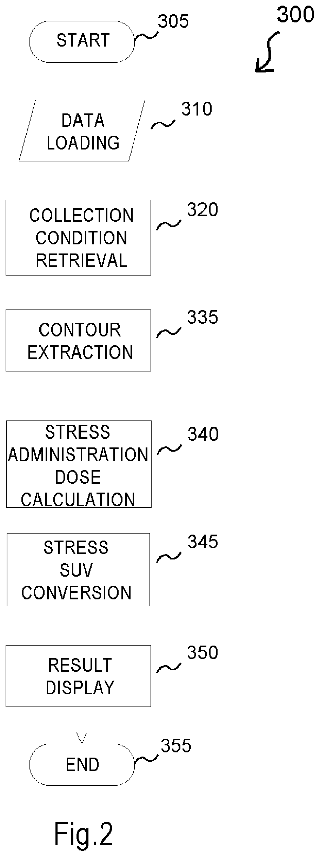

Next, the flow of SUV conversion processing 300 of nuclear medicine image data disclosed in the present application will be described with reference to FIG. 2. The processing 300 may be performed by the system 100 in which the SUV conversion program 120 is executed by the CPU 102. In some embodiments, midway through the processing 300, the contour extraction/volume calculation program 124 may be called from the SUV conversion program 120 and executed by the CPU 102 to perform certain processing.

Step 305 indicates the start of processing. In step 310, data to be processed by the SUV conversion program 120 is loaded. In other words, all or part of the image data 130 is read from the mass storage unit 106 and is stored in the main memory unit 104. The image data 130 may be directly imported from an external nuclear medicine apparatus into the main memory unit 104 via the network interface 109.

In step 320, various collection conditions of the image data 130 are retrieved. The various collection conditions include the following information, for example.

- A radiation dose measured before administration of a radiopharmaceutical agent to a subject (radiation dose before administration). For example, a value obtained by measuring the radiation dose of a whole administration syringe containing a radiopharmaceutical agent to be administered
- The measurement date and time of a radiation dose before administration
- The date and time at the start of data collection
- The data collection time
- A radiation dose measured after administration of the radiopharmaceutical agent to the subject (radiation dose after administration). For example, a measurement value of the radiation dose remaining in the syringe after administration
- The measurement date and time of a radiation dose after administration
- The half-life of a tracer contained in the radiopharmaceutical agent
- Becquerel calibration factor (BCF, a factor for converting a radiation count value into a radioactivity concentration (for example, Bq/ml))

In some embodiments, these collection conditions may be included in the image data 130. In such a case, the system 100 may read the information from the data 130 and store the information in the main memory unit 104 or the mass storage unit 106.

In some embodiments, the system 100 may be configured to create and display a user interface (for example, a dialog box) to which an operator inputs these collection conditions. When an operator inputs intended collection conditions, the system 100 may store these collection conditions in the main memory unit 104 or the mass storage unit 106.

In some embodiments, each pixel value of the image data 130 may represent a tissue radioactivity concentration. In such a case, the BCF is not used and thus is not required to be retrieved.

As described above, the system 100 may be configured to store the retrieved collection condition information in the main memory unit 104 or the mass storage unit 106. In the example, the collection condition information for the image data 130 is considered to be stored in the collection condition file 131.

In step 335, the image data 130 after alignment is subjected to myocardial contour extraction. The processing in step 335 may be performed through execution of the contour extraction/volume calculation program 124 by the CPU 102. As described above, some algorithms and software for myocardial contour extraction are known, and such an algorithm is disclosed by the present applicant in PCT International Publication (WO2013/047496A1), for example. The program instructions included in the contour extraction/volume calculation program 124 may be configured to use the algorithm to extract the myocardial contour.

In some embodiments, the contour extraction/volume calculation program 124 may be configured to use the extracted contour to calculate the myocardial volume. For example, the number of pixels present between the intima and the adventitia of the extracted myocardium may be multiplied by a pixel-volume conversion factor (for example, volume per pixel) to give a myocardial volume.

In step 340, the radiation dose administered to a subject is calculated. The information required for the calculation of an administered radiation dose is the following information.

- A radiation dose measured before administration of a radiopharmaceutical agent to a subject (radiation dose before administration)
- The measurement date and time of a radiation dose before administration
- The date and time at the start of data collection
- A radiation dose measured after administration of the radiopharmaceutical agent to the subject (radiation dose after administration)

- The measurement date and time of the radiation dose after administration
- The half-life of a tracer contained in the radiopharmaceutical agent

In the present example, the information is retrieved in step 320 and is stored in the collection condition file 131. The system 100 may thus retrieve the information from the collection condition file 131 in step 340.

Subsequently, an administered radiation dose is calculated in accordance with the following formulas:

```
Decay time 1 (seconds) = |Measurement date and time of
radiation dose before administration - Date and time at
start of data collection|

Decay time 2 (seconds) = |Measurement date and time of
radiation dose after administration - Date and time at
start of data collection|

Decay coefficient = LN (2.0) / Half-life (seconds) (LN:
natural logarithm to the base e)
```

```
Administered  radiation  dose  =  {Radiation  dose  before
administration × Exp (− Decay coefficient × Decay time 1)}
− {Radiation  dose  after  administration  ×  Exp  (−  Decay
coefficient × Decay time 2)}.
```

In step 345, the pixel value of each pixel in the image data 130 is converted into an SUV. The existing SUV conversion uses the body weight of a subject for normalization. In contrast, the SUV conversion of the present embodiment is performed in accordance with formula 1.

```
[Formula 1]
      SUV   =   Tissue  radioactivity  concentration  /
(Administered radiation dose / Myocardial weight)
```

Each parameter will be briefly described below.

Tissue radioactivity concentration: it may be a value after standardization or interpolation process, for example. Each pixel value of typical nuclear medicine image data represents either a tissue radioactivity concentration or a radiation count value. When the pixel value of each pixel in image data 130 represents a radioactivity concentration, the pixel value itself can be used as the tissue radioactivity concentration. When the pixel value of each pixel in image data 130 represents a radiation count value, the value is required to be multiplied by a becquerel calibration factor (BCF), which is a factor for converting a radiation count value into a radiodensity (for example, Bq/ml), to convert the pixel value into a radioactivity concentration. When the BCF is needed, the system may be configured to retrieve the conversion factor in step 320, for example.

Administered radiation dose: the administered radiation dose determined in step 340.

Myocardial weight: it is calculated on the basis of the myocardium contour data obtained in step 335. For example, the number of pixels presents between the intima and the adventitia of the extracted myocardium may be multiplied by a pixel-volume conversion factor to give a myocardial volume, and the myocardial volume may be multiplied by a myocardial volume-myocardial weight conversion factor (density factor) to give a myocardial weight. The density factor can be known literature data and may be 1.05, for example. The myocardial weight may be calculated in step 335 or in the present step. In some embodiments, the myocardial weight calculation algorithm may be installed in the contour extraction/volume calculation program 124 or in the SUV conversion program 120. The calculated myocardial weight may be stored in the main memory unit 102 or the mass storage unit 106. In some embodiments, the myocardial weight may be stored in a register of the CPU 102.

The BCF can be determined by a known method. For example, a nuclear medicine image of a vial (or a syringe) containing a radiopharmaceutical agent having a known total radioactivity can be taken, and the BCF can be calculated in accordance with the following formula:

```
      BCF  =  Decay-corrected  total  radioactivity  (Bq)  /
(Total count of all slices / Collection time (seconds)).
```

To determine the BCF from the data obtained using a cylindrical phantom, the following formulas can be used:

```
      Volume  factor  =  Average  count  value  per  slice  /
(Volume of single pixel × Collection time (seconds))
```

$$BCF = \text{Decay-corrected total radioactivity (Bq)} / (\text{Phantom volume} \times \text{Volume factor}).$$

In some embodiments, the BCF may be subjected to collection time correction. The collection time correction may be performed by multiplying {Volume of single pixel [cm$^3$] / Collection time [sec]} by BCF, for example.

The image data after conversion of the pixel value of each pixel into an SUV may be stored as SUV image data 150 in the mass storage unit 106, for example (see FIG. 1) .

In the present example, the weight of the myocardium in which a tracer is accumulated is used as a standard to normalize myocardial nuclear medicine image data. The normalized value thus reflects actual conditions of a cardiac function more correctly than in the related art, and myocardial blood flow conditions can be imaged more objectively. In other words, the validity and reliability are improved when pieces of data are compared between different measurement dates and times and between different subjects, for example.

In some embodiments, the myocardial volume may be used to perform normalization in place of the myocardial weight. Alternatively, another index relating to the heart size may be used to perform normalization.

In step 350, the calculation result of the increase rate of the myocardial blood flow is displayed. The display may be made in various manners. For example, when the SUV image data 150 storing the result is three-dimensional image data in which the pixel value of each pixel is converted into an SUV, the result may be displayed as brightness or color tone corresponding to the SUV at a position of the corresponding pixel on a short axis tomogram or a three-dimensional image.

In some embodiments, the nuclear medicine image data to be subjected to SUV conversion may be two-dimensional array data or a two-dimensional polar map in place of the three-dimensional data. In such a case, the SUV image data 150, which is also two-dimensional array data or a two-dimensional polar map, may be displayed.

The invention of the present application has been specifically described with reference to preferred examples. The description and the attached drawings are not intended to limit the scope of the invention of the present application, but are intended to satisfy the requirements of the law. Embodiments of the invention of the present application include various variations in addition to the above-exemplified embodiments. For example, various numerical values shown in the description or the drawings are illustrative values and are not intended to limit the scope of the invention. Individual features included in the various examples that have been described in the description or the drawings are not limited to usage with examples in which these features are explicitly explained to be included, but may be used in combination with other examples that have been described herein or various specific examples that have not been described. In particular, the processes presented in the flowcharts do not necessarily need to be performed in the described order. According to the preference of an executor, the processes may be performed in a changed order or in parallel, or as a plurality of blocks integrally implemented, or in a loop as appropriate. These variations are all included in the scope of the invention disclosed in the present application. The form of implementing processes does not limit the scope of the invention. The order of the description of the processes defined in the claims does not necessarily specify the mandatory order of the processes. For example, an embodiment specifying a different order of the processes and an embodiment that executes the processes in a loop are also included in the scope of the invention according to the claims.

For example, an embodiment of the SUV conversion program 120 can include a single program, a program group including a plurality of independent programs, and a program integrated with all or part of the contour extraction/volume calculation program 124. A program can be installed in various manners, which are well known, and all the various manners are included in the scope of the invention disclosed in the present application.

The novel SUV disclosed in the present application is characterized using an index relating to the size of a myocardium for normalization, and thus the SUV of the present application can be used in all the fields in which the normalization is appropriate, such as various nuclear medicine examinations of the heart.

**Claims**

1. A method (300) for processing myocardial nuclear medicine image data, comprising:

   operating an apparatus as first means for storing a heart parameter serving as a value relating to a size of a heart and as second means for storing an administered radiation dose; and
   converting (350) pixel values of at least part of pixels of the image data using the values stored in the first means and the second means into standardized uptake values (SUVs) in accordance with the following formula, and storing the SUVs:

$$\text{SUV} = \text{Tissue radioactivity concentration} / (\text{Administered radiation dose} / \text{Value based on heart parameter}).$$

2. The method according to claim 1, wherein the heart parameter is a myocardial weight, and the value based on the heart parameter is also a myocardial weight.

3. The method according to claim 1, wherein the heart parameter is a myocardial volume, and the value based on the heart parameter is a myocardial weight calculated by multiplying the myocardial volume by a conversion factor.

4. The method according to claim 1, wherein the tissue radioactivity concentration is a value obtained by multiplying the pixel value by a becquerel calibration factor (BCF).

5. The method according to claim 4, wherein the becquerel calibration factor is subjected to collection time correction.

6. The method according to claim 1, further comprising outputting the SUVs.

7. A computer program comprising a program instruction configured to cause an apparatus to perform the method as claimed in any one of claims 1 to 6 when the computer program is executed by processing means in the apparatus.

8. An apparatus comprising processing means and memory means, the memory means storing a program instruction, the program instruction being configured to perform the method as claimed in any one of claims 1 to 6 when the program instruction is executed by the processing means.

**Patentansprüche**

1. Verfahren (300) zum Verarbeiten myokardialer Nuklearmedizin-Bilddaten, umfassend:

Betreiben einer Vorrichtung als ein erstes Mittel zum Speichern eines Herzparameters, der als ein Wert dient, der sich auf eine Größe eines Herzes bezieht, und als ein zweites Mittel zum Speichern einer verabreichten Strahlendosis; und
Umwandeln (350) von Pixelwerten mindestens eines Teils von Pixeln der Bilddaten unter der Verwendung der in dem ersten Mittel und in dem zweiten Mittel gespeicherten Werte in SUVs (Standardized Uptake Values) gemäß der folgenden Formel und Speichern der SUVs:

$$\text{SUV} = \text{Gewebe-Radioaktivitäts-Konzentration} / (\text{verabreichte Strahlendosis} / \text{auf Herzparameter basierender Wert}).$$

2. Verfahren gemäß Anspruch 1, wobei der Herzparameter eine myokardiale Masse ist und der auf dem Herzparameter basierende Wert ebenfalls eine myokardiale Masse ist.

3. Verfahren gemäß Anspruch 1, wobei der Herzparameter ein myokardiales Volumen ist und der auf dem Herzparameter basierende Wert eine myokardiale Masse ist, die durch Multiplizieren des myokardialen Volumens mit einem Umrechnungsfaktor berechnet wird.

4. Verfahren gemäß Anspruch 1, wobei die Gewebe-Radioaktivitäts-Konzentration ein Wert ist, erhalten durch Multiplizieren des Pixelwerts mit einem Becquerel-Kalibrierungsfaktor (BCF).

5. Verfahren gemäß Anspruch 4, wobei der Becquerel-Kalibrierungsfaktor einer Erfassungszeitkorrektur unterzogen wird.

6. Verfahren gemäß Anspruch 1, ferner umfassend Ausgeben der SUVs.

**7.** Computerprogramm, umfassend einen Programmbefehl, konfiguriert, eine Vorrichtung zu veranlassen, das Verfahren gemäß einem der Ansprüche 1 bis 6 durchzuführen, wenn das Computerprogramm von einem Verarbeitungsmittel in der Vorrichtung ausgeführt wird.

**8.** Vorrichtung, umfassend ein Verarbeitungsmittel und ein Speichermittel, wobei in dem Speichermittel ein Programmbefehl gespeichert ist, wobei der Programmbefehl konfiguriert ist, das Verfahren gemäß einem der Ansprüche 1 bis 6 durchzuführen, wenn der Programmbefehl von dem Verarbeitungsmittel ausgeführt wird.

**Revendications**

**1.** Un procédé de traitement de données d'image de médecine nucléaire du myocarde, comprenant les étapes consistant à:

utiliser un appareil en tant que premier moyen pour stocker un paramètre cardiaque servant de valeur relative à la taille d'un coeur et en tant que second moyen pour stocker une dose de rayonnement administrée; et convertir les valeurs de pixel d'au moins une partie des pixels des données d'image en utilisant les valeurs stockées dans les premier et second moyens en valeurs d'absorption normalisées (SUV) conformément à la formule suivante, et mémoriser les SUV:

$$SUV = \text{Concentration de radioactivité du tissu/Dose de rayonnement administrée}$$

$$/\text{Valeur basée sur le paramètre cardiaque.}$$

**2.** Le procédé selon la revendication 1, dans lequel le paramètre cardiaque est le poids du myocarde, et la valeur relative au paramètre cardiaque est également le poids du myocarde.

**3.** Le procédé selon la revendication 1, dans lequel le paramètre cardiaque est un volume myocardique et la valeur basée sur le paramètre cardiaque est une masse myocardique calculée en multipliant le volume myocardique par un facteur de conversion.

**4.** Le procédé selon la revendication 1, dans lequel la concentration de radioactivité dans le tissu est une valeur obtenue en multipliant la valeur de pixel par un facteur de calibration de Becquerel (BCF).

**5.** Le procédé selon la revendication 4, dans lequel le facteur d'étalonnage en becquerel est soumis à une correction du temps de collecte.

**6.** Le procédé selon la revendication 1, comprenant en outre la sortie des valeurs SUV.

**7.** Un programme d'ordinateur comprenant des instructions de programme configurées pour amener un appareil à exécuter le procédé selon l'une quelconque des revendications 1 à 6, lorsque le programme d'ordinateur est exécuté par des moyens de traitement dans l'appareil.

**8.** Appareil comprenant un moyen de traitement et un moyen de mémoire, le moyen de mémoire stockant des instructions de programme, les instructions de programme étant configurées pour exécuter le procédé selon l'une quelconque des revendications 1 à 6, lorsque les instructions de programme sont exécutées par le moyen de traitement.

100

| 107 | DISPLAY I/F | | 102 | | 104 |
| 108 | PERIPHERAL I/F | | CPU | | RAM |
| 109 | NETWORK I/F | | | | |

106

| OS ~110 | STRESS DATA ~130 | ~131 |
| SUV CONVERSION PROGRAM ~120 | CONTOUR EXTRACTION/VOLUME CALCULATION PROGRAM ~124 | SUV ~150 |

Fig.1

Fig.2

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 5754718 B
- WO 2013047496A1 A

- WO 2013047496 A1

**Non-patent literature cited in the description**

- **YOSHIFUMI SUGAWARA ; KENNETH R. ZASAD-NY et al.** Reevaluation of the Standardized Uptake Value for FDG: Variations with Body Weight and Methods for Correction. *Radiology,* November 1999, vol. 213, 521-525

- **STEPHAN NEKOLLA et al.** The effect of different normalizations for the quantification of myocardial F-18 FDG uptake. *J Nucl Med,* 01 May 2015, vol. 56